# EUROPEAN PATENT SPECIFICATION

(11) **EP 2 814 405 B1**
(45) Date of publication and mention of the grant of the patent: **08.01.2020**
(21) Application number: 13705073.8
(22) Date of filing: 06.02.2013
(51) Int. Cl.: A61B 17/072

(54) **LINEAR STAPLER**
LINEARES HEFTGERÄT
AGRAFEUSE LINÉAIRE

(30) Priority: 14.02.2012 US 201261598395 P
(43) Date of publication of application: 24.12.2014
(73) Proprietor: Ethicon LLC, 00969 Guaynabo (PR)
(72) Inventor: NALAGATLA, Anil K., Mason, OH 45040 (US)
(74) Representative: Carpmaels & Ransford LLP
(86) International application number: PCT/US2013/024841
(87) International publication number: WO 2013/122792

(56) References cited:
- EP-A1- 0 373 762
- EP-A1- 2 090 239
- EP-A2- 0 514 139
- EP-A2- 0 754 433
- US-A- 4 508 253
- US-A- 5 735 445
- US-A1- 2005 143 759
- US-A1- 2011 295 242

## Description

### Technical Field

The present invention relates to a surgical stapling and cutting instrument. More specifically, the present invention relates to a linear stapler incorporating a cartridge with a lockout which can prevent a used or spent cartridge from refiring. The present invention also relates to a linear stapler incorporating a cartridge with a spent cartridge indicator which can indicate a spent cartridge.

### Background of the Invention

Surgical stapling and cutting instruments have been used in the prior art to simultaneously make an incision in tissue and apply lines of staples on opposing sides of the incision. End effectors of such instruments commonly include a pair of cooperating jaw members that, if the instrument is intended for endoscopic or laparoscopic applications, are capable of passing through a cannula passageway. One of the jaw members generally receives a staple cartridge having at least two laterally spaced rows of staples. The other jaw member defines an anvil having staple-forming pockets aligned with the rows of staples in the cartridge. The instrument includes a plurality of reciprocating wedges which, when driven distally, pass through openings in the staple cartridge and engage drivers supporting the staples to effect the firing of the staples toward the anvil.

It is often advantageous to build an end effector for the surgical stapler that is reusable. For instance, one patient may need a series of severing and stapling operations. Replacing an entire end effector for each operation tends to be economically inefficient. This is especially true if the end effector is built for strength and reliability over repeated operations. To that end, staple cartridges are fitted into the end effector prior to each operation of the surgical stapler.

While the staple cartridge containing staples provides numerous advantages, it is desirable to prevent inadvertent firing of the instrument when an unfired staple cartridge is not present. Otherwise, the severing of tissue may occur without the staples to minimize bleeding. It is particularly desirable that preventing such inadvertent firing be accomplished in a reliable way that is not subject to an intervening malfunction.

Moreover, for ease of manufacturing and assembly, it is further desirable that the lockout features be accomplished with a minimum number of components.

Consequently, significant needs exist for a linear stapler incorporating a cartridge with a lockout which can prevent a used or spent cartridge from refiring and for a linear stapler incorporating a cartridge with a spent cartridge indicator which can indicate a spent cartridge.

US4508253A discloses a surgical fastener applying apparatus including an actuator and a removable surgical fastener container cartridge. In the apparatus, the cartridge fits loosely into a cartridge holder part of the actuator which translates linearly toward or away from an anvil part of the actuator. As the cartridge holder translates toward the anvil, the cartridge is automatically brought into alignment and registration with the anvil.

EP0514139A2 discloses a stapler that includes a mechanism for sensing whether the stapler is loaded with a fired cartridge housing and for preventing the stapler from being closed or fired when loaded with the fired cartridge.

US20050143759A1 discloses a surgical instrument is adapted for applying a plurality of surgical fasteners to body tissue. The surgical instrument includes a frame having a proximal end and a distal end, with a handle positioned at the proximal end and an end effector positioned at the distal end. The end effector is shaped and dimensioned for supporting a cartridge module. A firing mechanism is associated with the end effector and the cartridge module for selective actuation. The end effector has a radius of curvature optimized for a male pelvis such that the end effector has a curvature of at most about (19.8cm) 7.8 inch diameter.

US5735445A discloses a surgical stapler having a supporting frame and a replaceable staple cartridge. The stapler provides a lockout feature for positively preventing refiring if the staple cartridge is spent. The lockout feature comprises a locking elementthat is connected to the approximation member and is adapted for movement to a position wherein the locking element blocks movement of the T-bar forwardly of the apparatus.

### Summary of the Invention

The present invention relates to a linear stapler incorporating a cartridge with a lockout which can prevent a used or spent cartridge from refiring, according to the appended set of claims.

### Brief Description Of The Drawings

Embodiments of the present invention will be described below with reference to the appended drawings, in which:
Figure 1 is a perspective view of a linear stapler in accordance with the embodiments f the present invention.
Figure 2 is a side view of the linear stapler in an unactuated open position.
Figure 3 is a side view of the linearstapler in an actuated closed position.
Figure 4 is a partial cross sectional view of an end effector of the linear stapler.
Figures 5A and 5B are respectively schematic view and sectional view of a staple river shown in Figure 4.
Figures 6A and 6B are respectively schematic view and sectional view of a staple cartridge shown in Figure 4.
Figure 7 is a view showing the relationship between the cartridge and the staple river before firing.
Figure 8 is a view showing the relationship between the cartridge and the staple river after firing.

### Embodiments

The invention is defined in the appended set of claims.

For convenience and ease of understanding, like parts are indicated by like reference signs in the context. It will be appreciated that the terms "proximal" and "distal" are used herein with reference to a user gripping a handle of an instrument. Thus, an end effector is distal with respect to a more proximal handle. It will be further appreciated that, for convenience and clarity, spatial terms such as "vertical" and " horizontal", "up" and " down" are used herein with respect to the drawings. However, surgical instruments are used in many orientations and positions, and these terms are not intended to be limiting and absolute.

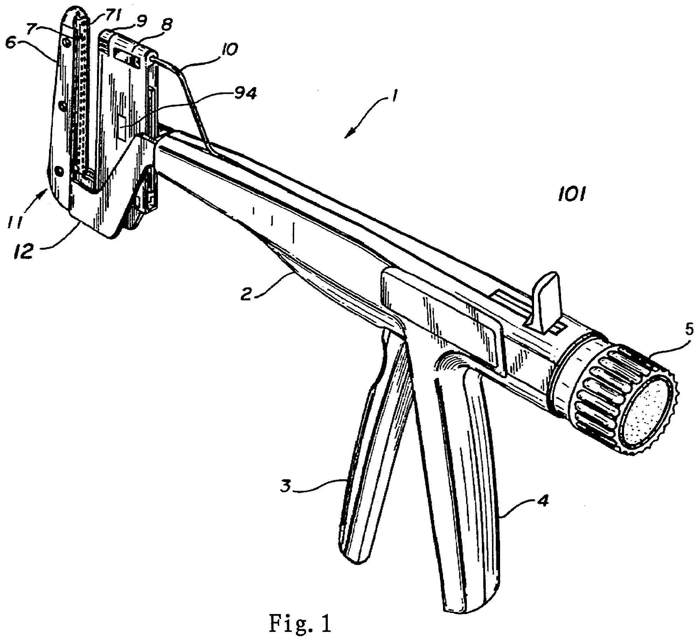

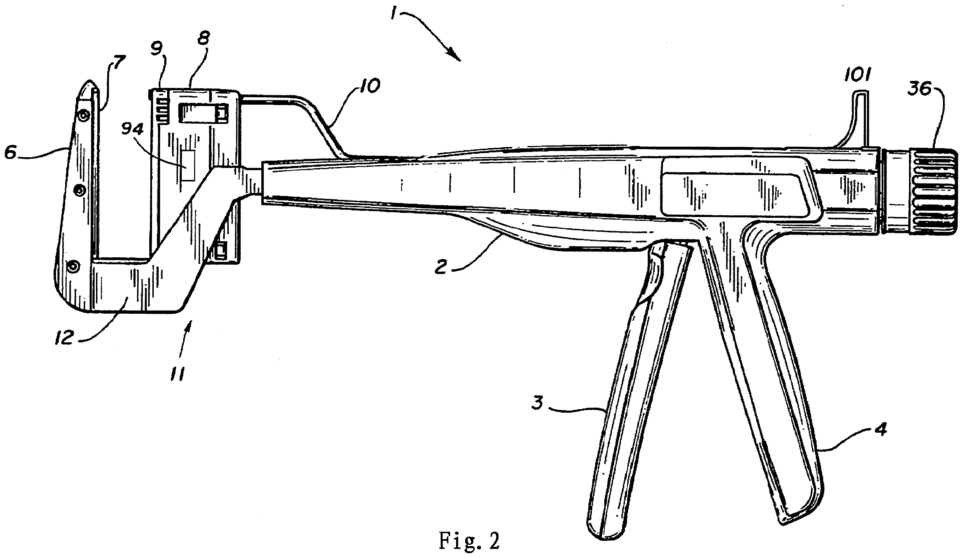

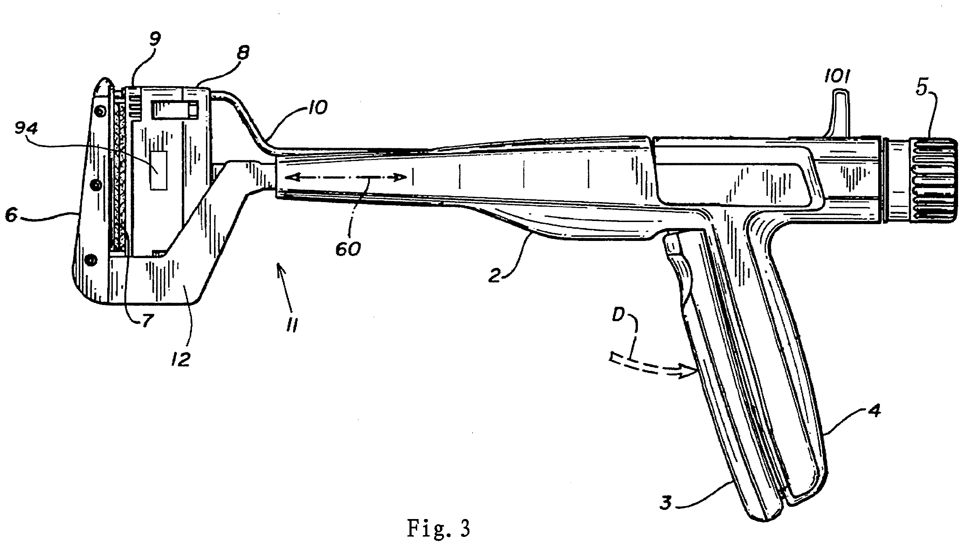

Figure 1 shows the perspective view of a stapler 1 according to the present invention. The stapler 1 comprises a support base 2 having a proximal end and a distal end, an end effector 11 located at the distal end of the support base 2, a trigger 3 and a handle 4 both located at a proximal side of the support base 2, and a knob 5 located at the proximal end of the support base 2. The end effector 11 is actuated and fired by the trigger 3 and the knob 5 so that the end effector 11 can staple the clamped tissue. Figure 2 shows the stapler 1 in an unactuated open position, and Figure 3 shows the stapler 1 in an actuated closed position with staples ejected to staple the tissue.

The components of the stapler 1 will be described in detail with reference to the appended drawings.

As shown in Figures 1 and 2, the end effector 11 is located at the distal end of the support base 2 and has a substantially U-shaped supporting structure 12 connected with the support base 2. The U-shaped supporting structure 12 is formed by extrusion, for example, of aluminum, with subsequent machining to create the supporting structure 12. In this way, multiple parts are not required and the associated cost of manufacture and assembly is substantially reduced. In addition, the overall stability may be enhanced and the stapler 1 is easy to be sterilized for cobalt irradiation will effectively penetrate the extruded aluminum. Moreover, less trauma to tissue based upon the smooth outer surface may be achieved via extrusion. The U-shaped supporting structure 12 supports a fixed jaw 6 and a movable jaw 8. The fixed jaw 6 in turn supports an anvil 7. The movable jaw 8 contains a cartridge 9 for accommodating surgical staples. A retaining pin 10 is supported on an upper portion of the support base 2. As shown in the figures, the retaining pin 10 extends through a through hole 43 and a through hole 27 in an upper end of the movable jaw 8. The retaining pin 10 is shiftable forwardly to a hole 71 in the anvil 7 to assure that the anvil 7 and the cartridge 9 are properly aligned and the tissue captured therebetween is appropriately maintained.

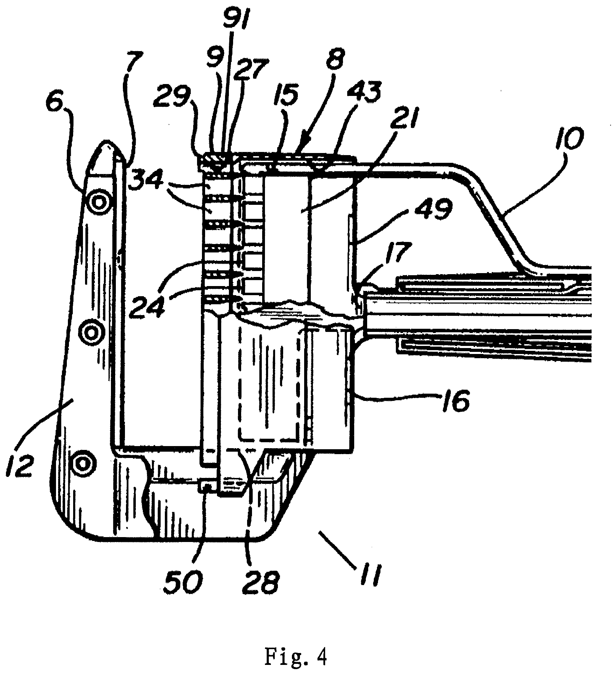

Reference is now made to Figure 4, wherein the movable jaw 8 and a cartridge assembly are illustrated in a cross-sectional view. The cartridge assembly comprises the cartridge 9 facing the anvil 7, a staple driver 15 proximal to the cartridge 9, a push rod 17 extending from the proximal side to the distal side, and a casing 16 surrounding the cartridge 9, the driver 15 and a part of the push rod 17. As shown in Figs. 1-3, the casing 16 is formed with an opening 94 to expose part of the cartridge 9. Detailed description of the parts is as follows.

As shown in Figure 4, the lower end of the cartridge 9 is formed with a slot 28 for guiding function. The distal surface of cartridge 9 (i.e., that surface facing the anvil) is provided with a forwardly extending spacer element 29 adjacent the through hole 27 and a forwardly extending spacer 50 adjacent the outermost end of the slot 28. These spacers cooperate with the anvil to determine the distalmost position of cartridge 9. Certainly, these elements are not absolutely necessary and can be omitted.

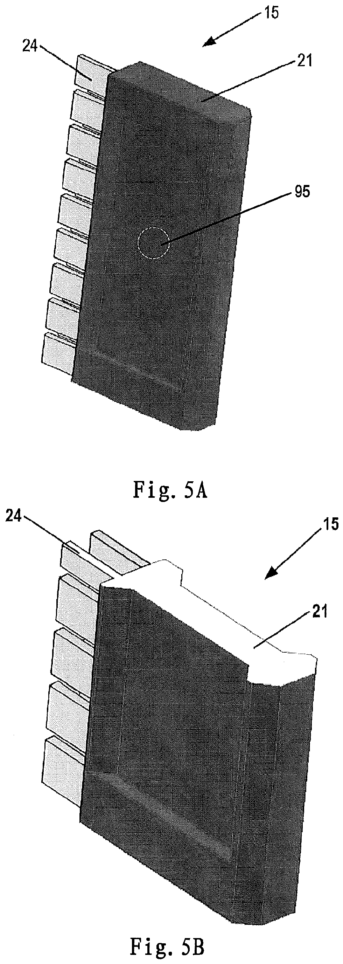

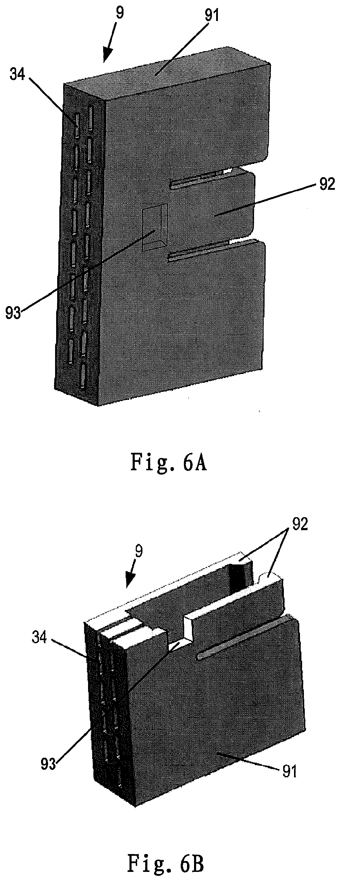

Figures 6A and 6B are respectively schematic view and sectional view of the staple cartridge according to an embodiment of the present invention, in which the spacer element and spacer are omitted for clarity. As shown in Figure 6A, the cartridge 9 comprises a general cuboid cartridge body 91 which may be made of polymer. The cartridge body 91 is formed with staple slots 34 in its distal end surface for accommodating staples. Each staple slot 34 is provided at its ends with additional grooves (not shown in the figures) intended to frictionally receive the legs of a surgical staple. The cartridge body 91 has an open proximal end for passage of the driver 15. On left and right side walls, the cartridge body 91 is provided with the cartridge lockout. The cartridge lockout can prevent a spent or used cartridge from refiring or reloading after being fired. Fig. 5A-8 also show a spent cartridge indicator which can indicate a spent cartridge.

As shown in Figure 6A, the cartridge lockout comprises deflecting features 92 provided on the left and right side walls of the cartridge body 91. Of course, those skilled in the art can conceive that the deflecting features 92 may be provided on upper and lower walls, or only provided on one of the walls of the cartridge. As shown in the Figures, the deflecting features 92 are strip-like elements extending proximally (i.e., towards the right side of the figure in Figure 6A or 6B) and each having at the proximal end thereof a protrusion protruding inward the cartridge. The other portion of the side wall of the cartridge body 91 has similar wall thickness. The deflecting features 92 may be polymer elements integral with the cartridge body 91 made by molding and the like, or separate metallic elements attached to the cartridge body 91 after being manufactured. Furthermore, the deflecting features 92 may be a leaf spring, a metallic wire having an additional protruding elastic tap at its proximal end, or a leaf spring having an enlarged proximal end, and the like. In brief, the deflecting features 92 may take any suitable forms as long as they can spring back into their original shapes when the retaining force forcing them into an open position is released. Certainly, those skilled in the art can appreciate that metallic deflecting features 92 may provide better stability and strength.

Also as shown in Fig. 6A, the spent cartridge indicator comprises an indicator window 93 formed in the cartridge body 91. In the embodiment shown in Fig. 6A, the indicator window 93 is located on an upper portion of the cartridge body 91. However, it should be appreciated that the indicator window 93 may be located at other positions, as long as the opening 94 of the casing 16 is arranged at a corresponding position to expose the indicator window 93. Alternatively, the casing 16 may be omitted or the casing 16 just encloses a part of the cartridge and does not cover the indicator window 93. Meanwhile, the shape of the indicator window 93 is not limited to the rectangular one as shown in the figures, it may be circular, square, triangular, quinquangular and so on.

Immediately proximal to the cartridge 9 is disposed the driver 15 (as shown in Figures 5A and 5B as two exemplary embodiments). The driver 15 has enough strength and is molded by appropriate plastic materials adapted to be used in a surgical environment, and preferably made from those plastic materials which can be sterilized by known methods. As shown in Figures 5A and 5B, the driver 15 is an integral, one-piece element comprising a driver body 21, a plurality of driving plates 24 extending forwardly from a distal surface of the driver body 21. The driving plates 24 are equal in number to the number of staples housed in the cartridge 9. The forwardly extending driving plates 24 are arranged in two parallel, spaced rows, with the driving plates of one row staggered with respect to the driving plates of the other, which corresponds to the arrangement of the staple slots 34 of the cartridge 9. As shown in Figure 5A, the driver body 21 is generally in a cuboid shape, with two proximal edges chamfered and left and right sides each having a recessed portion. As shown in the sectional view of the embodiment of Figure 5B, the section of the driver body 21 is in a general dog-bone shape. In one embodiment, the driver body 21 and driving plates 24 have different colors to serve as indicator features of the spent cartridge indicator. In another embodiment, the driver body 21 has a mark 95 (imaginary line in Fig. 5A) thereon as an indicator feature of the spent cartridge indicator. The mark 95 may be any marks which can draw attention, such as circular, square, triangular, cross shape marks and the like, or pocket, recess or small projection which does not affect movement known by those skilled in the art.

As shown in FIG. 4, the driving plates 24 of the driver 15 are insertable within the staple slots 34 of the cartridge 9. It will be appreciated that the tine 24 of the driver overlies a crown of a corresponding staple. In this way, when the driver 15 is shifted distally relative to the cartridge 9, the surgical staple will be ejected distally out of its staple slot 34.

Figure 4 also shows the casing 16. The casing 16, like the driver 15 and the cartridge 9, is an integral, one-piece, molded plastic member.

A proximal end of the push rod 17 (Figure 4) is associated with the trigger 3 and the knob 5 (Figure 1). Rotation of the knob 5 moves the push rod 17 distally and rotation of the trigger 3 towards the handle 4 (indicated by an arrow D in Figure 3) fires the cartridge 9, causing the push rod 17 to drive the driver 15 for forming the staples. A distal end of the push rod 17 terminates in a plate-like structure 49 (Figure 4). A distal end of the plate-like structure 49 may contact with the proximal end of the driver body 21 of the driver 15. When the push rod 17 is shifted in a distal direction, it will shift the driver 15 distally via the plate-like structure 49.

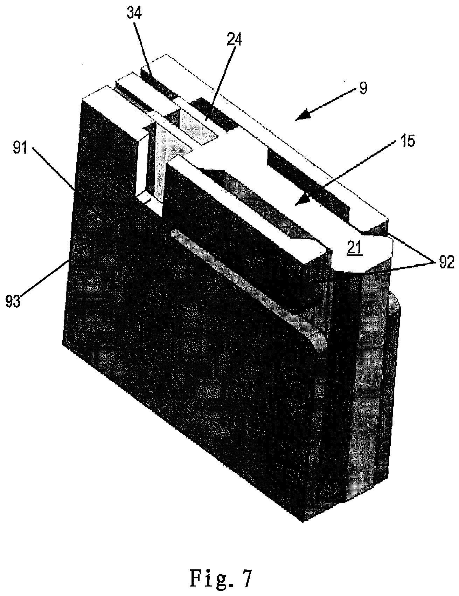

Figure 7 is a view showing the relationship between the cartridge and the staple driver before firing. As shown in Figure 7, the driving plates 24 of the driver 15 are just located at the proximal sides of the staple slots 34 of the cartridge 9, and the proximal end of the driver body 21 of the driver 15 does not pass the protrusions at the proximal ends of the deflecting features 92 yet. At this point, a part of the driving plate 24 can be seen through the indicator window 93 of the cartridge body 91.

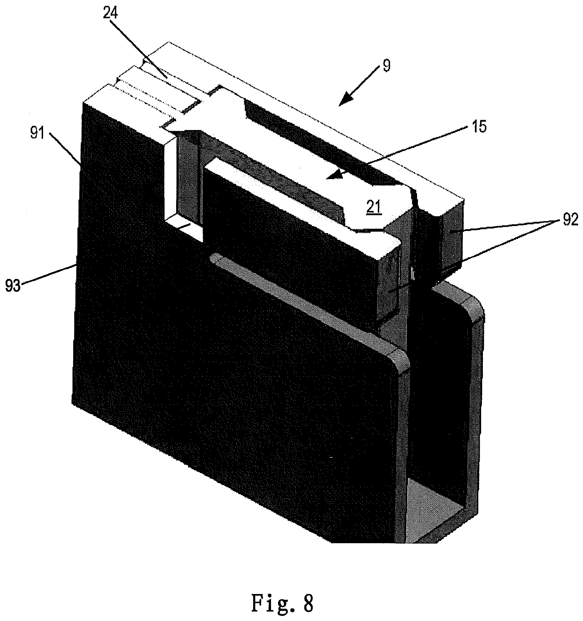

Figure 8 is a view showing the relationship between the cartridge and the staple driver after firing.

When the cartridge 9 is actuated by the push rod 17, the plate-like structure 49 of the push rod 17 contacts with the proximal end of the driver body 21 of the driver 15 and moves the driver 15 distally, the driving plates 24 of the driver 15 are further inserted into the staple slots 34 of the cartridge 9. As the driving plates 24 of the driver 15 are inserted into the staple slots 34, the protrusions of the deflecting features 92 slide on the proximal end of the driver body 21, are forced open and cannot spring back into the cartridge (meaning cannot go back to the cartridge's normal shape in which the deflecting features 92 are not deflected). After firing, as shown in Figure 8, the push rod 17 is retracted and the plate-like structure 49 is retracted together. At this point, the driving plates 24 of the driver 15 are kept in the staple slots 34 completely. The proximal end of the driver 15 passes the protrusions of the deflecting features 92 and enter the cartridge 9 completely. Then the proximal end of the driver body 21 of the driver 15 does not restrain the deflecting features 92 any more. The deflecting features 92 now springs back inward into the cartridge body 91, or at least spring back partially as long as the deflecting features come into position to stop the push rod 17 from being driven forwardly distally past them. As such, the deflecting features 92 stay between the plate-like structure 49 at the distal end of the push rod 17 and the driver 15. Therefore, when the user attempts to reload a used or spent cartridge, the deflecting features 92 will block the push rod 17 from being driven forwardly, thus preventing being refired.

As described above, the deflecting features may be provided on the upper and lower walls of the cartridge, or only provided on one of the left, right, upper and lower walls, as long as the proximal end of the driver body of the driver is provided with a corresponding structure so that the deflecting features are forced into an open position to abut against the outer side of the staple driver when the cartridge is not fired and springs back into the cartridge body's about regular dimension or shape after the staples in the cartridge are fired by the staple driver. The proximal end of the deflecting feature need not have a protrusion, but may have an enlarged portion at about the proximal end extending toward inside of the cartridge. For example, the enlarged portion can be a part that enlarges gradually from the distal to the proximal end enlarging in the direction extending toward the inside of the cartridge.

In the position shown in Fig. 8, the driving plates 24 of the driver 15 are kept in the staple slots 34 completely. Only a part of the driver body 21 can be seen through the indicator window 93 of the cartridge body 91. In the embodiment that the driver body 21 and the driving plates 24 have different colors, now the color shown in the indicator window 93 is different from that before firing the cartridge to indicate a user that the cartridge is fired. In the embodiment that the driver body 21 has a mark 95 thereon, the mark 95 can be seen through the indicator window 93 just at this moment to indicate a user that the cartridge is fired. Of course, the two manners may be combined so that when in the position shown in Fig. 8, not only the different color but also the mark can be seen through the indicator window 93 to more reliably indicate the fired cartridge. In other words, after the cartridge is fired, the indicator feature can be seen through the indicator window 93 to indicate a user that the cartridge is fired.

The various embodiments of the present invention have been described above in connection with linear staplers. It should be noted, however, that in other embodiments, the inventive surgical instrument disclosed herein need not to be a linear stapler, but may be a curved stapler and the like. The present invention also has application in conventional endoscopic and open surgical instrumentation as well as robotic-assisted surgery.

The instruments disclosed herein can be designed to be disposed of after a single use, or they can be designed to be used multiple times. In either case, however, the instrument can be reconditioned for reuse after at least one use. Reconditioning can include any combination of the steps of disassembly of the instrument, followed by cleaning or replacement of particular pieces, and subsequent reassembly. In particular, the instrument can be disassembled, and any number of the particular pieces or parts of the instrument can be selectively replaced or removed in any combination. Upon cleaning and/or replacement of particular parts, the instrument can be reassembled for subsequent use either at a reconditioning facility, or by a surgical team immediately prior to a surgical procedure. Those skilled in the art will appreciate that reconditioning of a instrument can utilize a variety of techniques for disassembly, cleaning/replacement, and reassembly. Use of such techniques, and the resulting reconditioned instrument, are all within the scope of the present application.

Although the present invention has been described herein in connection with certain disclosed embodiments, many modifications and variations to those embodiments may be implemented. For example, different types of end effectors may be employed. Also, where materials are disclosed for certain components, other materials may be used. The foregoing description and following claims are intended to cover all such modification and variations.

## Claims

1. A cartridge (9) for an end effector of a linear stapler, the cartridge (9) comprising:
a staple driver (15) comprising a proximal end and a distal end,
a cartridge body (91) including:
staple slots (34) for accommodating staples; and said cartridge being **characterized in that** it comprises
a cartridge lockout for preventing the cartridge from being re-fired once used or spent, the cartridge lockout comprising one or more deflecting features (92) arranged on a wall of the cartridge body (91) and extending proximally, wherein the deflecting features (92) and staple driver (15) are configured such that the deflecting features are forced into an open position by the proximal end of the staple driver to abut against an outer side of the staple driver (15) when the cartridge (9) is being fired and spring back toward an inside direction of the cartridge body (91) after the staples in the cartridge (9) are fired by the staple driver (15), such that the deflecting features (92) prevent the cartridge from being re-fired once being used or spent.

2. The cartridge (9) of claim 1, wherein the deflecting features (92) are integral with the cartridge body (91).

3. The cartridge (9) of claim 1, wherein the deflecting features (92) are attachable to the cartridge body (91).

4. The cartridge (9) of claim 1, wherein the deflecting features (92) are made of polymer or metal.

5. The cartridge (9) of claim 1, wherein the deflecting features (92) are leaf springs.

6. The cartridge (9) of claim 5, wherein the deflecting features (92) each comprise an enlarged proximal end.

7. A linear stapler (1) comprising:
a support base (2) having a proximal end and a distal end;
a trigger (3) located at the proximal end of the support base (2); and
an end effector (11) located at the distal end of the support base (2), configured to be fired by the trigger (3) and including the cartridge (9) of any preceding claim.

## Patentansprüche

1. Magazin (9) für einen Endeffektor eines linearen Klammergeräts, wobei das Magazin (9) Folgendes umfasst:
einen Klammertreiber (15), der ein proximales Ende und ein distales Ende umfasst,
einen Magazinkörper (91), der Folgendes umfasst:
Klammerschlitze (34) zur Aufnahme von Klammern; und
wobei das Magazin **dadurch gekennzeichnet ist, dass** es Folgendes umfasst:
eine Magazinsperre zum Verhindern des erneuten Auslösens des Magazins nach Gebrauch oder Verbrauch, wobei die Magazinsperre ein oder mehrere Biegemerkmale (92) umfasst, die an einer Wand des Magazinkörpers (91) angeordnet sind und sich proximal erstrecken, wobei die Biegemerkmale (92) und der Klammertreiber (15) derart konfiguriert sind, dass die Biegemerkmale durch das proximale Ende des Klammertreibers dahingehend in eine geöffnete Stellung gedrückt werden, an einer Außenseite des Klammertreibers (15) anzuliegen, wenn das Magazin (9) ausgelöst wird, und in einer nach innen verlaufenden Richtung des Magazinkörpers (91) zurückzuschnellen, nachdem die Klammern in dem Magazin (9) von dem Klammertreiber (15) herauskatapultiert wurden, so dass die Biegemerkmale (92) das erneute Auslösen des Magazins nach Gebrauch oder Verbrauch verhindern.

2. Magazin (9) nach Anspruch 1, wobei die Biegemerkmale (92) mit dem Magazinkörper (91) integral sind.

3. Magazin (9) nach Anspruch 1, wobei die Biegemerkmale (92) an dem Magazinkörper (91) anbringbar sind.

4. Magazin (9) nach Anspruch 1, wobei die Biegemerkmale (92) aus Polymer oder Metall hergestellt sind.

5. Magazin (9) nach Anspruch 1, wobei die Biegemerkmale (92) Blattfedern sind.

6. Magazin (9) nach Anspruch 5, wobei die Biegemerkmale (92) jeweils ein vergrößertes proximales Ende umfassen.

7. Lineares Klammergerät (1), das Folgendes umfasst:
eine Stützbasis (2) mit einem proximalen Ende und einem distalen Ende;
einen Auslöser (3), der an dem proximalen Ende der Stützbasis (2) positioniert ist; und
einen Endeffektor (11), der an dem distalen Ende der Stützbasis (2) positioniert ist, dazu konfiguriert ist, von dem Auslöser (3) ausgelöst zu werden, und das Magazin (9) nach einem vorhergehenden Anspruch umfasst.

## Revendications

1. Cartouche (9) pour un effecteur terminal d'une agrafeuse linéaire, la cartouche (9) comprenant :
un dispositif d'enfoncement d'agrafes (15) comprenant une extrémité proximale et une extrémité distale,
un corps de cartouche (91) comportant :
des fentes pour agrafes (34) pour recevoir des agrafes ; et ladite cartouche étant **caractérisée en ce qu'**elle comprend :
un verrouillage de cartouche pour empêcher que la cartouche ne soit activée à nouveau après qu'elle a été utilisée ou vidée, le verrouillage de cartouche comprenant un ou plusieurs éléments déflecteurs (92) disposés sur une paroi du corps de cartouche (91) et s'étendant dans la direction proximale, les éléments déflecteurs (92) et le dispositif d'enfoncement d'agrafes (15) étant configurés de telle sorte que les éléments déflecteur soient forcés dans une position ouverte par l'extrémité proximale du dispositif d'enfoncement d'agrafes de manière à buter contre un côté extérieur du dispositif d'enfoncement d'agrafes (15) lorsque la cartouche (9) est activée et à revenir dans une direction vers l'intérieur du corps de cartouche (91) après que les agrafes dans la cartouche (9) ont été projetées par le dispositif d'enfoncement d'agrafes (15), de telle sorte que les éléments déflecteurs (92) empêchent la cartouche d'être réactivée une fois utilisée ou vide.

2. Cartouche (9) selon la revendication 1, dans laquelle les éléments déflecteurs (92) sont intégrés au corps de cartouche (91).

3. Cartouche (9) selon la revendication 1, dans laquelle les éléments déflecteurs (92) peuvent être attachés au corps de cartouche (91).

4. Cartouche (9) selon la revendication 1, dans laquelle les éléments déflecteurs (92) sont fabriqués en polymère ou en métal.

5. Cartouche (9) selon la revendication 1, dans laquelle les éléments déflecteurs (92) sont des ressorts à lame.

6. Cartouche (9) selon la revendication 5, dans laquelle les éléments déflecteurs (92) comprennent chacun une extrémité proximale agrandie.

7. Agrafeuse linéaire (1), comprenant :
une base de support (2) ayant une extrémité proximale et une extrémité distale ;
une gâchette (3) située au niveau de l'extrémité proximale de la base de support (2) ; et
un effecteur terminal (11) situé au niveau de l'extrémité distale de la base de support (2),
configuré pour être déclenché par la gâchette (3) et comportant la cartouche (9) selon l'une quelconque des revendications précédentes.
